# EUROPÄISCHE PATENTANMELDUNG

(11) **EP 0 918 201 A1**
(43) Veröffentlichungstag der Anmeldung: **26.05.1999**
(21) Anmeldenummer: 98119675.1
(22) Anmeldetag: 17.10.1998
(51) Int. Cl.: F26B 5/06

(54) **Verfahren zum Befüllen einer Spritze sowie Vorrichtung zur Durchführung des Verfahrens**

(30) Priorität: 19.11.1997 DE 19751226
(71) Anmelder: Arzneimittel GmbH Apotheker Vetter & Co. Ravensburg, D-88212 Ravensburg (DE)
(72) Erfinder: Vetter, Helmut, 88212 Ravensburg (DE); Otto, Thomas, 88250 Ravensburg (DE)
(74) Vertreter: Dziewior, Joachim, Dipl.-Phys. Dr.

(57) **Zusammenfassung**

Das erfindungsgemäße Verfahren zum Befüllen einer Spritze, insbesondere einer Spritze dient für medizinische Zwecke, mit einer Substanz, die im in die Spritze eingefüllten Zustand einem volumenvermindernden Verfahrensschritt, insbesondere einer Lyophilisierung, unterworfen wird. Dazu wird zunächst an dem zu befüllenden Ende des Spritzenzylinders (1) ein zum Spritzenzylinder (1) hin offener und gegen diesen abgedichteter Füllbehälter (3) aufgesetzt. Dann wird die Substanz in den Füllbehälter (3) sowie den Spritzenzylinder (1) eingebracht und anschließend die den Spritzenzylinder (1) sowie den Füllbehalter (3) füllende Substanz dem volumenvermindernden Verfahrensschritt unterworfen. Schließlich wird der Füllbehälter (3) vom Spritzenzylinder (1) abgenommen und der Spritzenzylinder (1) geschlossen oder weiteren Prozeßschritten zugeführt.

## Beschreibung

Die Erfindung betrifft ein Verfahren zum Befüllen einer Spritze, insbesondere einer Spritze für medizinische Zwecke, die mit einer Substanz befüllt einem Lyophilisationsprozeß unterworfen wird, wobei die Befüllung im Vergleich zu ihrer gelösten Form am Ende des Prozesses eine Volumenverminderung aufweist.

Weiter betrifft die Erfindung eine Vorrichtung zur Durchführung dieses Verfahrans.

Bei der Herstellung von Spritzen, die mit pharmazeutischen Substanzen vorgefüllt sind, ist es oftmals erforderlich, die zunächst in gelöster Form vorliegenden Substanzen von ihrem Lösungsmittel zu befreien, um eine ausreichend lange Haltbarkeit der Substanz zu gewährleisten. Dies geschieht insbesondere durch die Lyophilisierung, wonach die Produkte in gefriergetrockenetem Zustand häufig eine erheblich höhere Stabilität und damit Haltbarkeit aufweisen. Diese lyophilisierten pharmazeutischen Produkte werden meist in sogenannten Doppelkammerspritzen untergebracht, wobei eine der beiden Kammern ein geeignetes Lösungsmittel enthält, das kurz vor der Applikation dem lyophilisierten Produkt wieder zugeführt wird. Gewöhnlich nimmt das Lyophilisat nach erfolgter Gefriertrocknung den selben Raum ein wie in gelöster Form.

Unter den Produkten, die lyophilisiert und vor der Applikation mittels eines Lösungsmittels rekonstituiert werden müssen, gibt es solche, die in ihrer lyophilisierten Form (als Lyophilisat) dem Patienten verabreicht werden. Darüber hinaus ist es für die spätere Anwendung solcher Substanzen wünschenswert, daß diese während des Gefriertrocknungsprozesses eine Volumenreduzierung erfahren, um so vom späteren Anwender gezielter appliziert werden zu können. Eine Volumenreduzierung hat jedoch beim Einsatz gewöhnlicher Injektionsgefäße die Folge, daß bei diesen Anwendungen nach erfolgter Lyophilisierung ein großes überschüssiges Volumen, daß zum Abfüllen des flüssigen Produktes erforderlich war, verbleibt.

Dieses Volumen nimmt daher einerseits einen überflüssig großen Raum ein und kann darüber hinaus für den späteren Anwender während der Applikation der Substanz hinderlich sein.

Der Erfindung liegt daher die Aufgabe zugrunde, ein Verfahren zum Befüllen einer Spritze sowie eine zu ihrer Durchführung geeignete Vorrichtung anzugeben, die es erlaubt, eine dem Volumen des lyophilisierten Produkts angepaßte Spritzengröße zu verwenden, so daß eine effektive Befüllung der Spritze möglich ist, wobei weiter die Möglichkeit bestehen soll, daß das Injektionssystem eine Öffnung mit maximaler Größe aufweist, also keine Verengung im (kanülenseitigen) Kopfbereich aufweist.

Diese Aufgabe wird in verfahrensmäßiger Hinsicht dadurch gelöst, daß an dem zu befüllenden Ende des Spritzenzylinders ein zum Spritzenzylinder hin offener und gegen diesen abgedichteter Füllbehälter aufgesetzt wird, daß dann die Substanz in den Füllbehälter sowie den Spritzenzylinder eingebracht und anschließend die den Spritzenzylinder sowie den Füllbehälter füllende Substanz dem volumenvermindernden Verfahrensschritt unterworfen wird, und daß schließlich der Füllbehälter vom Spritzenzylinder abgenommen und der Spritzenzylinder geschlossen oder weiteren Prozeßschritten zugeführt wird.

Der durch die Erfindung erreichte Vorteil besteht im wesentlichen darin, daß durch den Füllbehälter das effektive Volumen der Spritze vorübergehend vergrößert wird, wobei mit zunehmenden Fortschritt der Lyophilisierung, also geringer werdendem Anteil des Lösungsmittels die Substanz sich im Spritzenzylinder ansammelt, so daß am Ende der Lyophilisierung der Füllbehälter abgenommen und im übrigen erneut verwendet werden kann.

In vorrichtungsmäßiger Hinsicht wird die Aufgabe durch eine Spritze, insbesondere eine Spitze für medizinische Zwecke, mit einer Substanz, die im in die Spritze eingefüllten Zustand einen volumenvermindernden Verfahrensschritt, insbesondere einer Lyophilisierung, unterworfen wird, wobei die Spritze von einem Spritzenzylinder und einem darin angeordneten Stopfen gebildet ist, an dessen einem Ende ein Kanülenansatz- oder Verschlußteil und an dessen anderem Ende eine Fingerauflage mit Kolbenstange ansetzbar ist, gelöst durch einen Füllbehälter, der mit einem Anschlußteil an einem der beiden Enden des Spritzenzylinders aufsetzbar, dabei zum Spitzenzylinder hin offen und gegen diesen abgedichtet ist und der im auf den Spritzenzylinder aufgesetzten Zustand an seinem oberen Ende eine Einfüllöffnung für die Substanz aufweist.

In bevorzugter Ausführungsform der Erfindung ist das Anschlußteil des Füllbehälters von einer Zylinderhülse gebildet, die den Spritzenzylinder außenseitig formschlüssig umgreift.

Weiter ist es von Vorteil, wenn das Anschlußteil des Füllbehälters einen ins Innere des Spritzenzylinders vorstehenden Kragen aufweist, dessen zur Wand des Spritzenzylinders weisende Mantelfläche mit einer Aufnahmenut für eine Dichtung versehen ist. Hierdurch ist insbesondere sicher gestellt, daß der Bereich der Innenmantelfläche zwischen der Dichtung und der Stirnseite des Spritzenzylinders nicht mit dem Lösungsmittel bzw. dem Lyophilisat in Berührung kommt, da in diesem Bereich beim späteren Verschließen des Spritzenzylinders in der Regel Dichtungsstopfen oder ähnliches eingesetzt werden.

Im übrigen kann zweckmäßigerweise auch in die zwischen der Zylinderhülse und dem Kragen gebildete Ringnut eine Dichtung eingelegt sein.

Weiter ist es im Rahmen der Erfindung vorgesehen, daß die Zylinderhülse axial über den Kragen vorsteht.

Schließlich hat es sich als zweckmäßig herausgestellt, wenn der Füllbehälter gleiche Querschnittsgestalt und -abmessungen besitzt wie der Spritzenzylinder.

Im folgenden wird die Erfindung an einem in der Zeichnung dargestellten Ausführungsbeispiel näher erläutert; es zeigen:
- Fig. 1: einen Spritzenzylinder mit aufgesetztem Füllbehälter und eingefüllter, zu lyophilisierender Lösung,
- Fig. 2: den Gegenstand nach Fig. 1, jedoch nach erfolgter Lyophilisierung,
- Fig. 3: den nach Abschluß der Lyophilisierung verschlossenen und fertig konfektionierten Spritzenzylinder mit abgehobenem Füllbehälter,
- Fig. 4: die mit X gekennzeichnete Detaildarstellung nach Fig. 3.

Die in der Zeichnung dargestellte Vorrichtung dient zum Befüllen einer Spritze, insbesondere einer Spritze für medizinische Zwecke, mit einer Substanz, die nach ihren Einfüllen in die Spritze einem volumenvermindernden Verfahrensschritt unterworfen wird. Einen derartigen volumenvermindernden Verfahrensschritt stellt insbesondere die Lyophilisierung dar, bei welcher zunächst ein in einem flüssigen Lösungsmittel befindlicher Wirkstoff in den Spritzenzylinder gebracht und anschließend durch die Lyophilisierung das Lösungsmittel entzogen wird, so daß der Wirkstoff selbst anschließend in gefriergetrockneter Form vorliegt. Um hierbei aus den unterschiedlichsten Gründen zu vermeiden, daß im Anschluß an die Lyophilisierung ein großes freies Volumen im Spritzenzylinder 1 verbleibt, ist ein Füllbehälter 3 vorgesehen, der mit einem Anschlußteil 8 an einem der beiden Enden des Spritzenzylinders 1 aufsetzbar ist. In dem Ausführungsbeispiel ist vorgesehen, den Füllbehälter 3 am kanülenseitigen Ende des Spritzenzylinders 1 anzuordnen, wobei das andere Ende durch einen Stopfen 2 geschlossen ist.

Der Füllbehälter 3 ist dabei zum Spritzenzylinder 1 hin offen und gegen diesen abgedichtet. Im auf den Spritzenzylinder 1 aufgesetzten Zustand weist der Füllbehälter 3 an seinem oberen Ende eine Einfüllöffnung 9 für die Substanz auf.

Im einzelnen ist das Anschlußteil 8 des Füllbehälters 3 von einer Zylinderhülse 10 gebildet, die den Spritzenzylinder 1 außenseitig formschlüssig umgreift. Weiter ist das Anschlußteil 8 des Füllbehälters 3 mit einem ins Innere des Spritzenzylinders 1 vorstehendem Kragen 11 versehen, dessen zur Wand des Spritzenzylinders 1 weisende Mantelfläche mit einer Aufnahmenut für eine Dichtung 4 versehen ist. Diese Dichtung 4 ist insbesondere in der vergrößerten Darstellung nach Fig. 4 gut erkennbar. Diese Dichtung 4, die bei vollständig aufgesetztem Füllbehälter 3 mit Abstand von dem zu befüllenden Ende des Spritzenzylinders 1 angeordnet ist, sorgt darüber hinaus dafür, daß der an die Stirnseite des Spritzenzylinders 1 angrenzende Randbereich frei von Lösungsmittel bzw. Lyophilisat bleibt und somit eine geeignete saubere Dichtfläche für einen hier später einzusetzenden Stopfen 6 oder dergleichen bildet.

Darüber hinaus kann auch, wie dies wiederum aus Fig. 4 deutlich hervorgeht, in die zwischen der Zylinderhülse 10 und dem Kragen 11 gebildete Ringnut 12 eine weitere Dichtung 5 eingelegt sein.

Um dem Füllbehälter 3 einen ausreichenden Halt auf dem Spritzenzylinder 1 zu geben, steht die Zylinderhülse 10 axial über den Kragen 11 vor. Im übrigen besitzen der Füllbehälter 3 und der Spritzenzylinder 1 gleiche Querschnittsgestalt und Abmessungen, wodurch das obere Ende des Füllbehälters 3 in seiner Gestalt genau dem oberen Ende des Spritzenzylinders 1 selbst entspricht, wodurch bei Einsatz der Füllbehälter 3 keinerlei Anpassungen oder Änderungen der Befülleinrichtungen - außer einer Höhenanpassung - erforderlich sind.

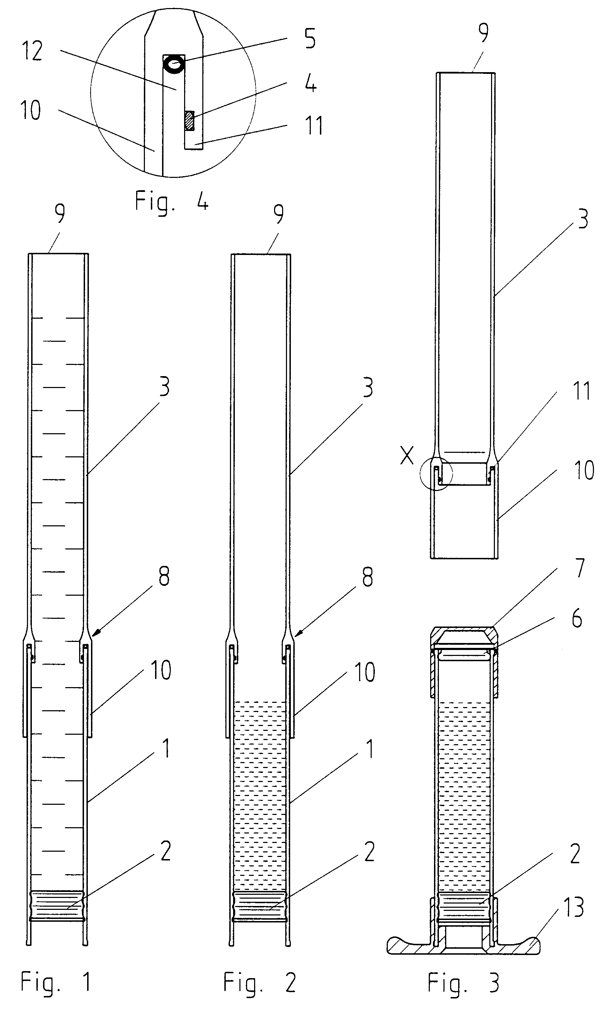

Die einzelnen, in den Fig. 1 bis 3 dargestellten Verfahrensschritte laufen so ab, daß zunächst der Füllbehälter 3 auf den Spritzenzylinder 1 aufgesetzt wird und sodann die Befüllung der sich in flüssiger Lösung befindlichen Substanz erfolgt, wobei sich die durch horizontale Striche in der Fig. 1 angedeutete Befüllung bis nahe zum oberen Rand des Füllbehälters 3 erstreckt.

Nach erfolgter Lyophilisierung befindet sich die durch kürzere horizontale Striche in Fig. 2 angedeutete trockene Substanz ausschließlich in Spritzenzylinder 1, so daß nun der Füllbehälter 3 abgenommen und der Spritzenzylinder 1 durch den Stopfen 6 und ein geeignetes Verschlußteil 7 geschlossen werden kann, wie dies in Fig. 3 dargestellt ist.

## Patentansprüche

1. Verfahren zum Befüllen einer Spritze, insbesondere einer Spritze für medizinische Zwecke, die mit einer Substanz befüllt einen Lyophilisationsprozeß unterworfen wird, wobei die Befüllung im Vergleich zu ihrer gelösten Form am Ende des Prozesses eine Volumenverminderung aufweist, dadurch gekennzeichnet, daß an dem zu befüllenden Ende des Spritzenzylinders (1) ein zum Spritzenzylinder (1) hin offener und gegen diesen abgedichteter Füllbehälter (3) aufgesetzt wird, daß dann die Substanz in den Füllbehälter (3) sowie den Spritzenzylinder (1) eingebracht und anschließend die den Spritzenzylinder (1) sowie den Füllbehälter (3) füllende Substanz dem volumenvermindernden Verfahrensschritt unterworfen wird, und daß schließlich der Füllbehälter (3) vom Spritzenzylinder (1) abgenommen und der Spritzenzylinder (1) geschlossen oder weiteren Prozeßschritten zugeführt wird.

2. Vorrichtung zum Befüllen einer Spritze gemäß Verfahren nach Anspruch 1, insbesondere einer Spritze für medizinische Zwecke, mit einer Substanz, die im in die Spritze eingefüllten Zustand einem volumenvermindernden Verfahrensschritt, insbesondere einer Lyophilisierung, unterworfen wird, wobei die Spritze von einem Spritzenzylinder (1) und einem darin angeordneten Stopfen (2) gebildet ist, an dessen einem Ende ein Kanülenansatz- oder Verschlußteil (7) und an dessen anderem Ende eine Fingerauflage (13) mit Kolbenstange ansetzbar ist, gekennzeichnet durch einen Füllbehälter (3), dar mit einem Anschlußteil (8) an einem der beiden Enden des Spritzenzylinders (1) aufsetzbar, dabei zum Spritzenzylinder (1) hin offen und gegen diesen abgedichtet ist und der im auf den Spritzenzylinder (1) aufgesetzten Zustand an seinem oberen Ende eine Einfüllöffnung (9) für die Substanz aufweist.

3. Vorrichtung nach Anspruch 2, dadurch gekennzeichnet, daß das Anschlußteil des Füllbehälters (3) von einer Zylinderhülse (10) gebildet ist, die den Spritzenzylinder (1) außenssitig formschlüssig umgreift.

4. Vorrichtung nach Anspruch 2 oder 3, dadurch gekennzeichnet, daß das Anschlußteil (8) des Füllbehälters (3) einen ins Innere des Spritzenzylinders (1) vorstehenden Kragen (11) aufweist, dessen zur Wand des Spritzenzylinders (1) weisende Mantelfläche mit einer Aufnahmenut für eine Dichtung (4) versehen ist.

5. Vorrichtung nach Anspruch 4, dadurch gekennzeichnet, daß in die zwischen der Zylinderhülse (10) und dem Kragen (11) gebildete Ringnut (12) eine Dichtung (5) eingelegt ist.

6. Vorrichtung nach Anspruch 4 oder 5, dadurch gekennzeichnet, daß die Zylinderhülse (10) axial über den Kragen (11) vorsteht.

7. Vorrichtung nach einem der Ansprüche 2 bis 6, dadurch gekennzeichnet, daß der Füllbehälter (3) gleiche Querschnittsgestalt und -abmessungen besitzt wie der Spritzenzylinder (1).
